# EUROPEAN PATENT APPLICATION

(11) **EP 1 552 818 A1**
(43) Date of publication of application: **13.07.2005**
(21) Application number: 05250003.0
(22) Date of filing: 04.01.2005
(51) Int. Cl.: A61K 9/16, A61K 9/20

(54) **Release sustaining composition and sustained release preparation**

(30) Priority: 07.01.2004 JP 2004002296
(71) Applicant: SHIN-ETSU CHEMICAL CO., LTD., Chiyoda-ku, Tokyo (JP)
(72) Inventor: Hayakawa, Kazuhisa, Shin-Etsu Chemical Co., Ltd., Kubiki-ku Joetsu-shi Niigata-ken (JP); Fukui, Ikuo, Shin-Etsu Chemical Co., Ltd., Kubiki-ku Joetsu-shi Niigata-ken (JP)
(74) Representative: Stoner, Gerard Patrick

(57) **Abstract**

A release sustaining composition comprising a polyalkylene polyol derivative comprising repeating units U-1 derived from a water-soluble polyalkylene polyol and a polyisocyanate and repeating units U-2 derived from a dihydroxy compound and a polyisocyanate in a molar ratio of U-1/U-2 between 0.5/0.5 and 0.99/0.01 and optionally a water-soluble cellulose ether is combined with an active ingredient to make a sustained release preparation.

## Description

### TECHNICAL FIELD

This invention relates to a release sustaining composition and a sustained release preparation.

### BACKGROUND

Prior art known methods of sustaining release of active ingredients include the microcapsulation of active liquid with encapsulants and the impregnation of adsorptive materials with active liquid. These methods have been utilized in a variety of fields including pharmaceutical drugs, health foods, perfumes, agricultural chemicals and the like.

Suitable shell materials that accommodate or cover the active liquid include natural polymers such as gelatin and cellulose derivatives and synthetic polymers such as polyamides. Gelatin is the most common among currently commercialized matrix materials. The traditional source for gelatin is cattle, which is nowadays suspected of potential infection with the mad cow disease. There is an increasing need for a replacement for gelatin.

For example, JP-A 63-287543 discloses a method of making microcapsules by using as a shell material at least one polymer selected from among polyvinyl acetal diethylaminoacetate, hydroxypropyl methyl cellulose phthalate, hydroxypropyl methyl cellulose acetate phthalate, and the like, dissolving the polymer in an organic solvent such as ethanol, adding an aqueous liquid thereto, optionally adding an acid or base, followed by coacervation (or phase separation). Since this method uses an organic solvent, there are left concerns about the presence of residual organic solvent in microcapsules and the safety in operation.

It was thus proposed to form a shell material from two types of polymers through coacervation as disclosed in JP-A 2000-33259. Microcapsules are prepared by mixing under alkaline conditions an aqueous solution of hydroxypropyl methyl cellulose phthalate or hydroxypropyl methyl cellulose acetate succinate with an aqueous solution of a methacrylic acid copolymer in an equally electrically charged state, effecting coacervation, and adding a separating agent such as sodium chloride. Regrettably the coating having two different types of polymers mixed suffers from incompatibility and does not always provide stable sustained release.

An object of the present invention is to provide a release sustaining composition which is capable of providing stable sustained release without requiring gelatin and organic solvents which are unwanted for safety; and a sustained release preparation using the same.

We have discovered that a release sustaining composition comprising a specific polyalkylene polyol derivative obtained from a comb-shaped diol can form a release controllable coat having safety and high stability; and that a sustained release preparation using the composition allows for stable sustained release of active ingredients.

In one aspect, the invention provides a release sustaining composition comprising a polyalkylene polyol derivative comprising repeating units U-1 having the general formula (1) and repeating units U-2 having the general formula (2) in a molar ratio of U-1/U-2 between 0.5/0.5 and 0.99/0.01. 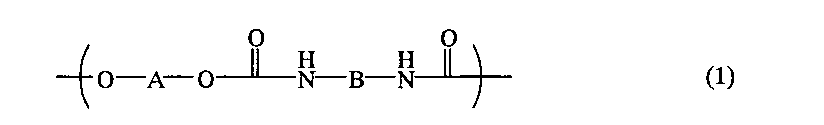 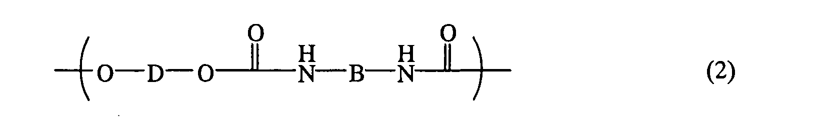 Herein A is a divalent polyoxyalkylene residue derived from a water-soluble polyalkylene polyol, represented by HO-A-OH, having hydroxyl groups at least at both ends and a number average molecular weight of 400 to 100,000. B is a divalent organic residue derived from a polyisocyanate, represented by OCN-B-NCO, having 3 to 18 carbon atoms in total. D is a divalent organic residue derived from a dihydroxy compound selected from the following general formulae (3) to (5). 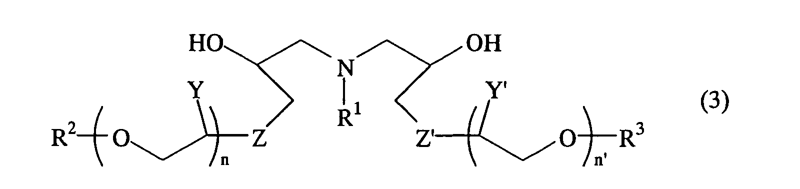 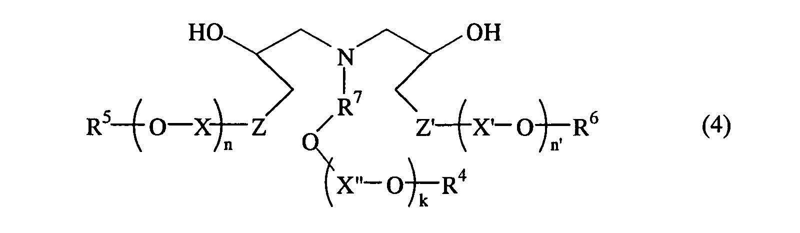 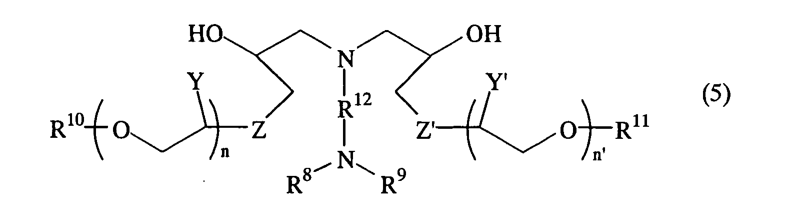 Herein each of R¹ and R⁴ is an unsubstituted or halo-substituted monovalent C₁-C₂₀ hydrocarbon group, each of R², R³, R⁵ and R⁶ , which may be the same or different, is an unsubstituted or halo-substituted monovalent C₄-C₂₁ hydrocarbon group, each of Y and Y', which may be the same or different, is hydrogen, methyl or CHCl₂, each of Z and Z' is an oxygen atom, sulfur atom or CH₂ group, n is an integer of 0 to 15 when Z is an oxygen atom and equal to 0 when Z is a sulfur atom or CH₂ group, n' is an integer of 0 to 15 when Z' is an oxygen atom and equal to 0 when Z' is a sulfur atom or CH₂ group, n and n' may be the same or different, each of X, X' and X", which may be the same or different, is a C₂-C₁₀ alkylene group, R⁷ is a C₂-C₁₀ alkylene group, k is an integer of 0 to 15, R⁸ and R⁹, which may be the same or different, are monovalent hydrocarbon groups, with a total number of carbon atoms being in a range of 2 to 20, each of R¹⁰ and R¹¹, which may be the same or different, is a monovalent C₄-C₂₁ hydrocarbon group, and R¹² is a C₂-C₇ alkylene group.

In a preferred embodiment, the dihydroxy compound having the general formula (3) or (4) is a dihydroxy compound having the general formula (6) or (7). 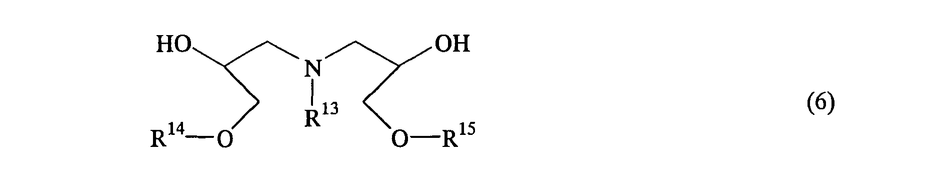 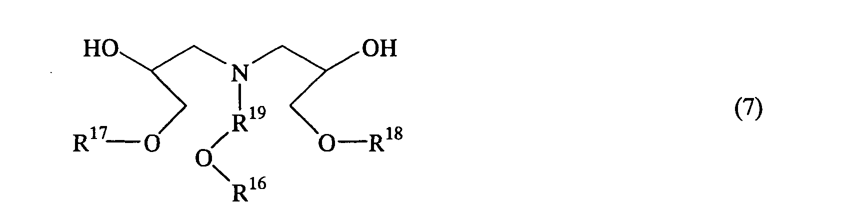 Herein R¹³ is a straight or branched C₄-C₁₈ alkyl group, each of R¹⁴ and R¹⁵, which may be the same or different, is a straight or branched C₄-C₁₈ alkyl group, R¹⁶ is a C₁-C₁₈ alkyl group, each of R¹⁷ and R¹⁸ which are the same is an unsubstituted or halo-substituted monovalent C₄-C₂₁ hydrocarbon group, and R¹⁹ is a 1,2-ethylene, 1,3-propylene or 1,4-butylene group.

The release sustaining composition may further comprise a water-soluble cellulose ether. The water-soluble cellulose ether is typically an alkyl cellulose or hydroxyalkyl alkyl cellulose.

Other aspects of the invention are the use of polyalkylene polyol derivatives as described herein in or as part of sustained release formulations, or for the purpose of controlling the release rate of another component incorporated with them in a solid preparation; methods of making controlled release preparations including or adapted to include at least one ingredient whose release rate is to be controlled in combination with the release sustaining composition, and controlled or sustained release preparations, especially solid particulate preparations such as tablets and granules.

The polyalkylene polyol derivative enables a release controllable coat having safety and high stability. In addition, the sustained release preparation using the composition and an active ingredient allows the active ingredient to release slowly over time in a stable manner.

### FURTHER EXPLANATIONS; OPTIONS AND PREFERENCES

As used herein, the notation (Cₙ-Cₘ) means a group containing from n to m carbon atoms per group.

In the release sustaining composition of the invention, a polyalkylene polyol derivative is used comprising repeating units having the general formulae (1) and (2). Provided that U-1 and U-2 denote the repeating units having formulae (1) and (2), respectively, the molar ratio of U-1/U-2 should fall in the range between 0.5/0.5 and 0.99/0.01.

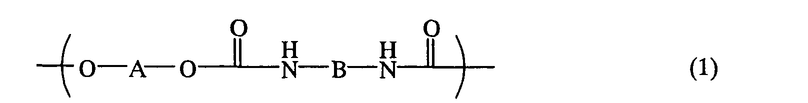

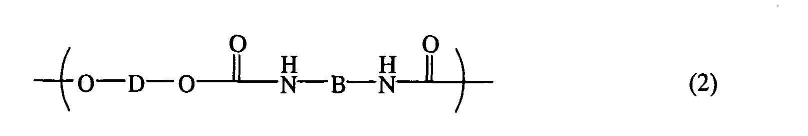

In formula (1), A is a divalent polyoxyalkylene residue derived from a water-soluble polyalkylene polyol, represented by HO-A-OH, having hydroxyl groups at least at both ends and a number average molecular weight of 400 to 100,000, preferably 500 to 80,000, and more preferably 1,000 to 50,000. The water-soluble polyalkylene polyol is an alkylene oxide polymer having hydroxyl groups at least at both ends, and examples of the alkylene oxide include ethylene oxide, propylene oxide, butylene oxide, epichlorohydrin, pentylene oxide, and hexylene oxide. These alkylene oxides may be used alone or in combination of two or more. It is noted that the number average molecular weight is a measurement by GPC-MALLS method.

In formulae (1) and (2), B is a divalent organic residue derived from a polyisocyanate, represented by OCN-B-NCO, in which the total number of carbon atoms is from 3 to 18, preferably from 3 to 16, and more preferably from 4 to 15. The polyisocyanate used herein may be suitably selected from linear or cyclic aliphatic polyisocyanates and aromatic polyisocyanates.

Suitable linear aliphatic diisocyanates include methylene diisocyanate, ethylene diisocyanate, trimethylene diisocyanate, 1-methylethylene diisocyanate, tetramethylene diisocyanate, pentamethylene diisocyanate, 2-methylbutane-1,4-diisocyanate, hexamethylene diisocyanate (HDI), heptamethylene diisocyanate, 2,2'-dimethylpentane-1,5-diisocyanate, lysine diisocyanate methyl ester (LDI), octamethylene diisocyanate, 2,5-dimethylhexane-1,6-diisocyanate, 2,2,4-trimethylpentane-1,5-diisocyanate, nonamethyl diisocyanate, 2,4,4-trimethylhexane-1,6-diisocyanate, decamethylene diisocyanate, undecamethylene diisocyanate, dodecamethylene diisocyanate, tridecamethylene diisocyanate, tetradecamethylene diisocyanate, pentadecamethylene diisocyanate, hexadecamethylene diisocyanate, trimethylhexamethylene diisocyanate, etc.

Suitable cyclic aliphatic polyisocyanates include cyclohexane-1,2-diisocyanate, cyclohexane-1,3-diisocyanate, cyclohexane-1,4-diisocyanate, 1-methylcyclohexane-2,4-diisocyanate, 1-methylcyclohexane-2,6-diisocyanate, 1-ethylcyclohexane-2,4-diisocyanate, 4,5-dimethylcyclohexane-1,3-diisocyanate, 1,2-dimethylcyclohexane-ω,ω'-diisocyanate, 1,4-dimethylcyclohexane-ω,ω'-diisocyanate, isophorone diisocyanate (IPDI), dicyclohexylmethane-4,4'-diisocyanate, dicyclohexylmethylmethane-4,4'-diisocyanate, dicyclohexyldimethylmethane-4,4'-diisocyanate, 2,2'-dimethyldicyclohexylmethane-4,4'-diisocyanate, 3,3'-dimethyldicyclohexylmethane-4,4'-diisocyanate, 4,4'-methylene-bis(isocyanatocyclohexane), isopropylidene bis(4-cyclohexylisocyanate) (IPCI), 1,3-bis(isocyanatomethyl)cyclohexane, hydrogenated tolylene diisocyanate (HTDI), hydrogenated 4,4'-diphenylmethane diisocyanate (HMDI), hydrogenated xylylene diisocyanate (HXDI), norbornane diisocyanatomethyl (NBDI), etc.

Suitable aromatic polyisocyanates include 1,3- and 1,4-phenylene diisocyanate, 1-methyl-2,4-phenylene diisocyanate (2,4-TDI), 1-methyl-2,6-phenylene diisocyanate (2,6-TDI), 1-methyl-2,5-phenylene diisocyanate, 1-methyl-3,5-phenylene diisocyanate, 1-ethyl-2,4-phenylene diisocyanate, 1-isopropyl-2,4-phenylene diisocyanate, 1,3-dimethyl-2,4-phenylene diisocyanate, 1,3-dimethyl-4,6-phenylene diisocyanate, 1,4-dimethyl-2,5-phenylene diisocyanate, m-xylene diisocyanate, diethylbenzene diisocyanate, diisopropylbenzene diisocyanate, 1-methyl-3,5-diethylbenzene-2,4-diisocyanate, 3-methyl-1,5-diethylbenzene-2,4-diisocyanate, 1,3,5-triethylbenzene-2,4-diisocyanate, naphthalene-1,4-diisocyanate, naphthalene-1,5-diisocyanate, 1-methylnaphthalene-1,5-diisocyanate, naphthalene-2,6-diisocyanate, naphthalene-2,7-diisocyanate, 1,1-dinaphthyl-2,2'-diisocyanate, biphenyl-2,4'-diisocyanate, biphenyl-4,4'-diisocyanate, 1,3-bis(1-isocyanato-1-methylethyl)benzene, 3,3'-dimethylbiphenyl-4,4'-diisocyanate, diphenylmethane-4,4'-diisocyanate (MDI), diphenylmethane-2,2'-diisocyanate, diphenylmethane-2,4'-diisocyanate, xylylene diisocyanate (XDI), etc.

In formula (2), D is a divalent organic residue derived from a dihydroxy compound selected from the following general formulae (3) to (5).

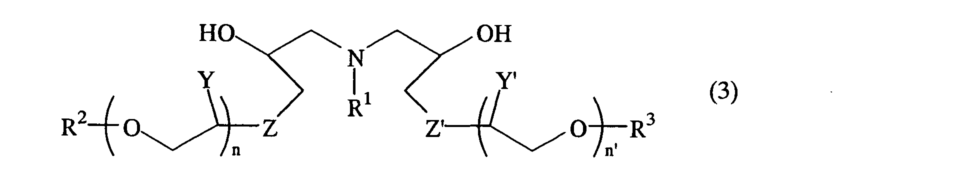

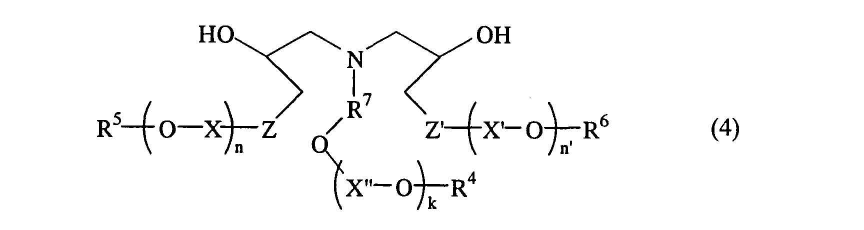

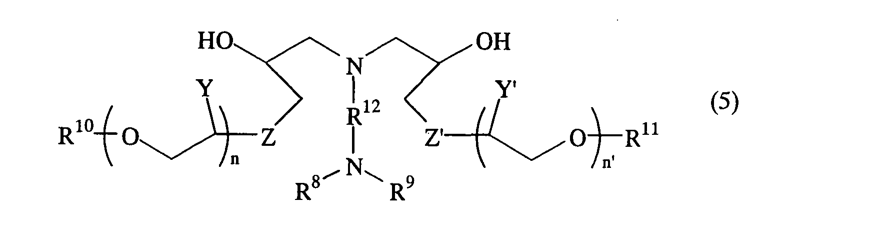

In the formulae, each of R¹ and R⁴ is an unsubstituted or halo-substituted monovalent C₁-C₂₀, preferably C₂-C₁₈, hydrocarbon group. Each of R², R³, R⁵ and R⁶, which may be the same or different, is an unsubstituted or halo-substituted monovalent C₄-C₂₁, preferably C₅-C₁₈, hydrocarbon group. Each of Y and Y', which may be the same or different, is hydrogen, methyl or CHCl₂. Each of Z and Z' is an oxygen atom, sulfur atom or CH₂ group. The subscript n is an integer of 0 to 15, preferably 2 to 12 when Z is an oxygen atom and equal to 0 when Z is a sulfur atom or CH₂ group; n' is an integer of 0 to 15, preferably 4 to 8 when Z' is an oxygen atom and equal to 0 when Z' is a sulfur atom or CH₂ group, and n and n' may be the same or different. Each of X, X' and X", which may be the same or different, is a C₂-C₁₀, preferably C₄-C₈, alkylene group. R⁷ is a C₂-C₁₀, preferably C₄-C₈, alkylene group. The subscript k is an integer of 0 to 15, preferably 2 to 12. R⁸ and R⁹, which may be the same or different, are monovalent hydrocarbon groups, with a total number of carbon atoms being in a range of 2 to 20, preferably 2 to 16. Each of R¹⁰ and R¹¹, which may be the same or different, is a monovalent C₄-C₂₁, preferably C₄-C₁₈, hydrocarbon group. R¹² is a C₂-C₇, preferably C₃-C₉, alkylene group.

Examples of unsubstituted or halo-substituted monovalent C₁-C₂₀ hydrocarbon groups include alkyl groups such as methyl, ethyl, propyl, butyl and pentyl, and haloalkyl groups such as bromobutyl and bromopropyl. Examples of unsubstituted or halo-substituted monovalent C₄-C₂₁ hydrocarbon groups include those groups of 4 to 20 carbon atoms exemplified above for the unsubstituted or halo-substituted monovalent C₁-C₂₀ hydrocarbon groups as well as hexyl and cyclohexyl groups.

Examples of C₂-C₁₀ alkylene groups include ethylene, propylene, butylene, amylene and hexylene. Examples of C₂-C₇ alkylene groups include those groups of 2 to 7 carbon atoms among the groups exemplified above for the C₂-C₁₀ alkylene groups. Exemplary combinations of monovalent hydrocarbon groups, in which the total number of carbon atoms is in a range of 2 to 20, include combinations of methyl with methyl, methyl with ethyl, methyl with propyl, and methyl with butyl.

Of the dihydroxy compounds having formula (3) or (4), dihydroxy compounds having formula (6) or (7) are preferred for their reactivity in polyisocyanate formation.

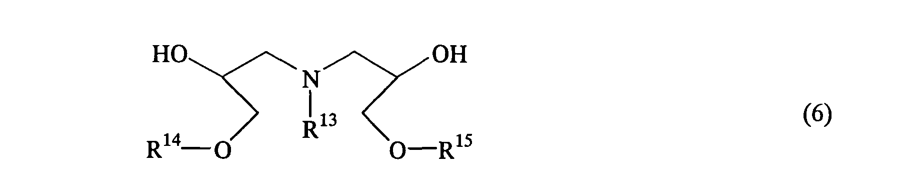

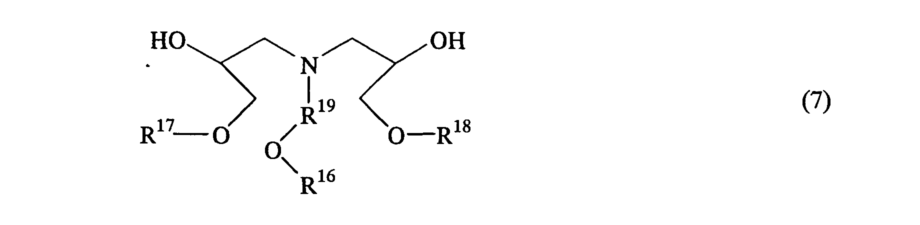

Herein R¹³ is a straight or branched C₄-C₁₈, preferably C₄-C₁₂, alkyl group. Each of R¹⁴ and R¹⁵, which may be the same or different, is a straight or branched C₄-C₁₈, preferably C₄-C₁₂, alkyl group. R¹⁶ is a C₁-C₁₈, preferably C₁-C₁₀, alkyl group. Each of R¹⁷ and R¹⁸, which are the same, is an unsubstituted or halo-substituted monovalent C₄-C₂₁, preferably C₄-C₁₆ , hydrocarbon group. R¹⁹ is a 1,2-ethylene, 1,3-propylene or 1,4-butylene group.

Examples of straight or branched C₄-C₁₈ alkyl groups include 2-methyl-butyl and 2,3-dimethyl-butyl. Examples of C₁-C₁₈ alkyl groups include methyl, ethyl, propyl, butyl, pentyl, hexyl, nonyl, etc. Examples of monovalent C₄-C₂₁ hydrocarbon groups are as exemplified above for the unsubstituted or halo-substituted monovalent C₄-C₂₁ hydrocarbon group.

In the polyalkylene polyol derivative comprising repeating units U-1 of formula (1) and repeating units U-2 of formula (2), the molar ratio of units U-1 to units U-2 is in the range between 0.5/0.5 and 0.99/0.01. Preferably it is at least 0.6/0.4. Preferably it is not above 0.8/0.2. If this ratio is too low, the polyalkylene polyol derivative may become less viscous and lose gel properties. If this ratio exceeds the upper limit, the derivative may become less dissolvable in water.

The polyalkylene polyol derivative has a weight average molecular weight (Mw) which is not particularly limited, but is preferably of the order from 10⁴ to 10⁶, more preferably falls in a range of about 10,000 to about 300,000 for conferring an appropriate viscoelasticity to a release sustaining composition containing the same. It is noted that the weight average molecular weight (Mw) is measurable by gel permeation chromatography (GPC) using polyethylene oxide or pullulan as the standard molecular weight substance.

Also preferably, the polyalkylene polyol derivative has a viscosity of 50 to 100,000 mPa·s, more preferably 100 to 100,000 mPa·s, as measured in a 2 wt% aqueous solution at 20°C by a BH type viscometer at 10 rpm, for conferring the desired sustained release effect to a sustained release preparation.

Illustrative examples of the polyalkylene polyol derivative include those compounds described as mortar thickener in JP-A 2001-348256.

The polyalkylene polyol derivative can, of course, be used alone since it becomes gel in water to provide a sustained release effect. However, when the composition is processed into a preparation, the integrity of the gel in gastric juice or water is so weak that the gel will be dissolved in gastric juice or water, indicating the possibility that the sustained release effect of the gel will not last for the desired period of time.

For this reason, it is recommended to add a water-soluble cellulose ether to the release sustaining composition for the purpose of stabilizing the sustained release effect of a coat made of the release sustaining composition.

The water-soluble cellulose ether used herein is not particularly limited. For conferring a good sustained release effect to the sustained release preparation, alkyl celluloses and hydroxyalkyl alkyl celluloses are preferred. Useful examples include methyl cellulose, hydroxypropyl methyl cellulose, hydroxyethyl methyl cellulose, and hydroxyethyl ethyl cellulose. They may be used alone or in admixture of two or more.

Preferably, the water-soluble cellulose ether has a viscosity of 50 to 100,000 mPa·s, more preferably 100 to 100,000 mPa·s, as measured in a 2 wt% aqueous solution at 20°C by a BH type viscometer at 10 rpm, for conferring the desired chemical leach-out to a sustained release preparation.

In the release sustaining composition, the water-soluble cellulose ether and the polyalkylene polyol derivative are typically blended in a weight ratio between 1/99 and 40/60, and preferably between 10/90 and 30/70. If the amount of the water-soluble cellulose ether is more than the range, the sustained release preparation may have a very slow initial leach-out. If the amount of the polyalkylene polyol derivative is more than the range, the sustained release stability may be reduced or lost as mentioned.

In the embodiment wherein the water-soluble cellulose ether and the polyalkylene polyol derivative are used together, the total amount of these components in the release sustaining composition is preferably 3 to 50% by weight, and more preferably 5 to 30% by weight, based on the total weight of the composition, for improving sustained release stability.

In the release sustaining composition, polyhydric alcohols, surfactants or the like may be compounded for the purpose of controlling the sustained release effect, typically in an amount of 1 to 20% by weight of the overall composition. Also, the release sustaining composition may be treated with an acid such as hydrochloric acid, acetic acid or phosphoric acid for improving the hardness and solubility of the composition.

The sustained release preparation of the invention is made from the release sustaining composition described above and an active ingredient to be slowly released, for example, by adding an active ingredient to a release sustaining composition comprising a polyalkylene polyol derivative and optionally a water-soluble cellulose ether, and pressing the composition into a preparation, or by admixing an aqueous solution comprising a polyalkylene polyol derivative and a water-soluble cellulose ether with an adsorbent, processing the mixture into granules, drying and impregnating the granules with an active ingredient.

The sustained release preparation may take the form of tablets and granules, but is not limited thereto. Tablets may have a diameter of about 5 to 15 mm. Typical granules are spherical or cylindrical and may have an average diameter of about 1 to 5 mm.

Suitable adsorbents used in the manufacture of preparations include powdery inorganic adsorbents such as bentonite, talc, and kaolin clay, and powdery organic polymers such as styrene, ethylene and acrylic polymers. Any suitable adsorbent that enables penetration and adsorption of the active ingredient to be slowly released may be selected.

The active ingredient to be slowly released may be selected from among pharmaceutical drugs, edible ingredients, forage ingredients, and agricultural chemicals. Examples include fat-soluble vitamins such as vitamin A, D, E and K, water-soluble vitamins such as vitamin C, polyethylene glycol dispersions, saccharides, amino acids, lactic acid bacteria such as bifidus, and pheromones.

Substantially water-insoluble pharmaceutical drugs are also useful as the active ingredient. By the term "substantially water-insoluble drugs" as used herein, it is meant that drugs are less absorptive on oral administration and classified as "substantially insoluble" or "very difficultly soluble" according to the Japanese Pharmacopoeia. The definition of solubility, as prescribed by the Japanese Pharmacopoeia, refers to the degree of dissolution within 30 minutes when a drug is admitted into a solvent, after grinding into powder if the drug is solid, and shaken at 20±5°C for 30 seconds every 5 minutes. The drug is rated as "substantially insoluble" when the volume of solvent (herein water) required to dissolve 1 g or 1 ml of the drug is 10,000 ml or more, and "very difficultly soluble" when the volume of solvent required to dissolve 1 g or 1 ml of the drug is from 1,000 ml to less than 10,000 ml. Exemplary drugs include nifedipine, phenacetin, phenytoin, digitoxin, nilvadipine, diazepam, griseofulvin, chloramphenicol, etc.

### EXAMPLE

Examples of the invention are given below together with Comparative Examples, by way of illustration and not by way of limitation.

### 1. Synthesis of dihydroxy compounds

### Synthesis Example 1

### Synthesis of dihydroxy compound #1

A 500-ml round bottom flask equipped with a magnetic stirrer, thermometer and dropping funnel was charged with 64.6 g of 2-ethylhexylamine (Kanto Chemical Co., Ltd.), with its interior being purged with nitrogen. The flask was heated at 60°C on an oil bath. With stirring, 220.0 g of 2-ethylhexyl glycidyl ether (Asahi Denka Co., Ltd., Adeka Glycirol ED518, epoxy number 220) from the dropping funnel was added dropwise over 40 minutes. After the addition was completed, the temperature of the oil bath was raised to 80°C, and the flask was heated at the temperature for 10 hours. Thereafter, the temperature of the oil bath was raised to 120°C, at which using a vacuum pump, a minor amount of unreacted reactants was distilled off under a vacuum of 400 Pa. There was obtained a dihydroxy compound #1 having 2 moles of 2-ethylhexyl glycidyl ether added per mole of 2-ethylhexylamine (an average molecular weight of 532 calculated from its OH value) in a yield of 90%.

### Synthesis Example 2

### Synthesis of dihydroxy compound #2

A 500-ml round bottom flask equipped with a magnetic stirrer, thermometer and dropping funnel was charged with 93.7 g of 3-(dodecyloxy)-1-propylamine (Koei Chemical Industry Co., Ltd.), with its interior being purged with nitrogen. The flask was heated at 60°C on an oil bath. With stirring, 188.0 g of 2-ethylhexyl glycidyl ether (Nagase Chemical Industry Co., Ltd., Denacol EX-121, epoxy number 188) from the dropping funnel was added dropwise over 40 minutes. After the addition was completed, the temperature of the oil bath was raised to 80°C, and the flask was heated at the temperature for 10 hours. Thereafter, the temperature of the oil bath was raised to 120°C, at which using a vacuum pump, a minor amount of unreacted reactants was distilled off under a vacuum of 400 Pa. There was obtained a dihydroxy compound #2 having 2 moles of 2-ethylhexyl glycidyl ether added per mole of 3-(dodecyloxy)-1-propylamine (an average molecular weight of 620 calculated from its OH value) in a yield of 85%.

### 2. Synthesis of polyalkylene polyol derivatives

### Synthesis Example 3

### Synthesis of polyalkylene polyol derivative #1

A 1000-ml separable flask of stainless steel was charged with 200 g of commercially available polyethylene glycol (PEG#6000, Sanyo Chemical Industry Co., Ltd., number average molecular weight 8,630), which was melted at 150°C in a nitrogen seal. With stirring, the melt was dried under a vacuum of 400 Pa for 3 hours. The residual water content was found to be 200 ppm.

The flask temperature was lowered to 70°C, after which the flask was filled with nitrogen at 101 kPa. BHT (di-tert-butylhydroxytoluene), 300 ppm, was added as an antioxidant. With stirring, 1.70 g of dihydroxy compound #1 of Synthesis Example 1 and 4.35 g of hexamethylene diisocyanate (Tokyo Chemical Industry Co., Ltd.) were fed to the flask (NCO/OH = 0.98 mol/mol). On addition of 0.05 g of DBTDL as a catalyst, a rapid viscosity buildup occurred in about 10 minutes. With stirring interrupted, the mixture was allowed to react at 70°C for 2 hours. The temperature was then raised to 120°C, at which the mixture was kept for 30 minutes. There was obtained a polyalkylene polyol derivative #1 having a melting point of about 60°C.

### Synthesis Example 4

### Synthesis of polyalkylene polyol derivative #2

A 1000-ml separable flask of stainless steel was charged with 200 g of polyethylene glycol (PEG#6000, Sanyo Chemical Industry Co., Ltd., number average molecular weight 8,630), which was melted at 150°C in a nitrogen seal. With stirring, the melt was dried under a vacuum of 400 Pa for 3 hours. The residual water content was found to be 200 ppm.

The flask temperature was lowered to 70°C, after which the flask was filled with nitrogen at 103 kPa. BHT (di-tert-butylhydroxytoluene), 300 ppm, was added as an antioxidant. With stirring, 1.90 g of dihydroxy compound #1 of Synthesis Example 1 and 4.41 g of hexamethylene diisocyanate (Tokyo Chemical Industry Co., Ltd.) were fed to the flask (NCO/OH = 0.98 mol/mol). On addition of 0.05 g of DBTDL as a catalyst, a rapid viscosity buildup occurred in about 10 minutes. With stirring interrupted, the mixture was allowed to react at 70°C for 2 hours. The temperature was then raised to 120°C, at which the mixture was kept for 30 minutes. There was obtained a polyalkylene polyol derivative #2 having a melting point of about 60°C.

### Synthesis Example 5

### Synthesis of polyalkylene polyol derivative #3

A 1000-ml separable flask of stainless steel was charged with 200 g of polyethylene glycol (PEG#6000, Sanyo Chemical Industry Co., Ltd., number average molecular weight 8,630), which was melted at 150°C in a nitrogen seal. With stirring, the melt was dried under a vacuum of 400 Pa for 3 hours. The residual water content was found to be 200 ppm.

The flask temperature was lowered to 70°C, after which the flask was filled with nitrogen at 103 kPa. BHT (di-tert-butylhydroxytoluene), 300 ppm, was added as an antioxidant. With stirring, 1.208 g of dihydroxy compound #2 of Synthesis Example 2 and 3.995 g of hexamethylene diisocyanate (Tokyo Chemical Industry Co., Ltd.) were fed to the flask (NCO/OH = 0.995 mol/mol). On addition of 0.05 g of DBTDL as a catalyst, a rapid viscosity buildup occurred in about 10 minutes. With stirring interrupted, the mixture was allowed to react at 70°C for 2 hours. The temperature was raised to 120°C, at which the mixture was kept for 30 minutes. There was obtained a polyalkylene polyol derivative #3 having a melting point of about 60°C.

### Synthesis Example 6

### Synthesis of polyalkylene polyol derivative #4

A polyalkylene polyol derivative #4 was obtained as in Synthesis Example 5 aside from changing the amounts of dihydroxy compound #2 and hexamethylene diisocyanate so as to provide a dihydroxy compound #2/polyethylene glycol ratio of 0.80 (% by weight) and a NCO/OH molar ratio of 0.995.

### Synthesis Example 7

### Synthesis of polyalkylene polyol derivative #5

A polyalkylene polyol derivative #5 was obtained as in Synthesis Example 5 aside from changing the amounts of dihydroxy compound #2 and hexamethylene diisocyanate so as to provide a dihydroxy compound #2/polyethylene glycol ratio of 0.50 (% by weight) and a NCO/OH molar ratio of 0.995.

For the polyalkylene polyol derivatives obtained in Synthesis Examples 3 to 7, their dihydroxy compound/polyethylene glycol ratio (% by weight), NCO/OH molar ratio, U-1/U-2 molar ratio, weight average molecular weight (Mw), and viscosity of a 2 wt% aqueous solution are shown in Table 1.

**Table 1**

| Polyalkylene polyol derivative | Dihydroxy compound/PEG (wt% of PEG) | NCO/OH (mol/mol) | U-1/U-2 (molar ratio) | Mw | Viscosity of 2 wt% aqueous solution (mPa·s) |
|---|---|---|---|---|---|
| #1 | 0.85 | 0.980 | 0.83/0.17 | 10,000 | 11,000 |
| #2 | 0.95 | 0.980 | 0.93/0.07 | 12,000 | 49,000 |
| #3 | 0.60 | 0.995 | 0.60/0.40 | 180,000 | 70,000 |
| #4 | 0.80 | 0.995 | 0.80/0.20 | 300,000 | 300,000 |
| #5 | 0.50 | 0.995 | 0.50/0.50 | 250,000 | 130,000 |

In Table 1, the Mw and the viscosity of 2 wt% aqueous solution were measured as follows.

### (1) Weight average molecular weight

Mw was measured using a gel permeation chromatograph GPC-MALLS (Showa Denko K.K.) and pullulan standards under the following conditions.

Detector: DAWN-DSP, Shodex RI-71, both Showa Denko K.K.

Column: Shodex SB-806MHQ, Showa Denko K.K.

Solvent: 0.1M NaNO₃

Temperature: 40°C

### (2) Viscosity of 2 wt% aqueous solution

For each polyoxyalkylene polyol derivative, a 2 wt% aqueous solution was prepared and its viscosity at 20°C was measured using a BH type viscometer at 10 rpm.

### Examples 1 to 8

### Sustained release compositions

The polyalkylene polyol derivatives shown in Table 1 and the water-soluble cellulose ethers having different Mw (all available from Shin-Etsu Chemical Co., Ltd.) shown in Table 2 were used in the combinations shown in Table 3. Specifically, 50 g of the polyalkylene polyol derivative was combined optionally with 50 g of a 2 wt% aqueous solution of the water-soluble cellulose ether and then with 50 g of bentonite (Kunibond, Kunimine Industries Co., Ltd.). The mixture was admitted into a 2-liter vessel of a homogenizer (Nippon Seiki Co., Ltd.) which was operated at 1,000 rpm for agitation. The mixture was granulated into granules having a diameter of about 1 to 5 mm, which were dried in a fluidized bed dryer (Midget Dryer) at an outlet air temperature of 100°C. The granules were sieved through a screen having an opening of 5 mm for removing coarse granules, obtaining sized granules of the release sustaining composition.

### Comparative Example 1

Granules were prepared as in Example 1 without using the polyalkylene polyol derivative and the water-soluble cellulose ether, and using only water instead. They were similarly dried and sieved, obtaining sized granules.

**Table 2**

| Water-soluble cellulose ether | | Methoxy content (mol%) | Hydroxypropoxyl content (mol%) | Mw | Viscosity of 2 wt% aqueous solution (mPa·s) |
|---|---|---|---|---|---|
| Designation | Type (trade name) | | | | |
| A | HPMC (60SH-100) | 29.6 | 8.8 | 120,000 | 95 |
| B | HPMC (60SH-4000) | 29.5 | 9.0 | 400,000 | 4800 |
| C | HPMC (90SH-100) | 23.9 | 7.2 | 120,000 | 107 |
| D | MC (SM-100) | 30.0 | - | 130,000 | 105 |
| HPMC: hydroxypropyl methyl cellulose | | | | | |
| MC: methyl cellulose | | | | | |

In Table 2, the Mw of cellulose ether was measured under the same conditions as described above. The viscosity of a 2 wt% aqueous solution of cellulose ether was measured under the same conditions as described above using a BH type viscometer.

**Table 3**

| | Polyalkylene polyol derivative | Water-soluble cellulose ether |
|---|---|---|
| Example 1 | 1 | - |
| Example 2 | 1 | A |
| Example 3 | 1 | B |
| Example 4 | 2 | B |
| Example 5 | 3 | C |
| Example 6 | 4 | C |
| Example 7 | 5 | D |
| Example 8 | 1 | - |
| Comparative Example 1 | - | - |

### Examples 9-16, Comparative Example 2

### Sustained release preparations

The granules obtained in Examples 1 to 8 and Comparative Example 1 were dipped in a vitamin C liquid for one hour, and dried in a dryer at 40°C, obtaining dry vitamin C-containing granules as sustained release preparations.

A granule sample was put in artificial gastric juice (35°C) as prescribed in the Japanese Pharmacopoeia, 14th Edition. With stirring, vitamin C leached out of the granules into the gastric juice. A change of yellow color due to leached vitamin C was periodically monitored by a color meter (Suga Tester Co., Ltd.). For the sustained release preparations of Examples 9 to 16, the propensity that the development of yellow color gradually increased over 6 hours was seen. For the preparation of Comparative Example 2, the granules disintegrated immediately after the admission into gastric juice, with no slow release being found. Table 4 shows the time it takes the yellow color to cease from changing.

**Table 4**

| | Yellow color change end time (hr) |
|---|---|
| Example 9 | 5 |
| Example 10 | 7 |
| Example 11 | 8 |
| Example 12 | 6 |
| Example 13 | 5 |
| Example 14 | 4 |
| Example 15 | 4 |
| Example 16 | 6 |
| Comparative Example 2 | 0.5 |

### Example 17

To 50 parts by weight of acetaminophen was added 50 parts by weight of polyalkylene polyol derivative #1 in Table 1 in powder form (average particle diameter 60 µm). From the mixture, tablets having a diameter of 10 mm and a thickness of 5 mm were made using a continuous tableting machine (Kikusui Mfg. Co., Ltd.). By the test of measuring the leach-out rate in artificial gastric juice as prescribed in the Japanese Pharmacopoeia, 14th Edition, the time it took the ingredient to cease from leaching out was found to be 4 hours.

Note that the average particle diameter was measured by a full automatic rotating/tapping particle size distribution meter (Seishin Enterprise Co., Ltd.).

### Example 18

Polyalkylene polyol derivative #1 in Table 1 in powder form (average particle diameter 60 µm) and hydroxypropyl methyl cellulose were mixed in a weight ratio of 1:1 to form a release sustaining composition. Using this composition, tablets were made as in Example 17. In the leach-out test, the time it took the ingredient to cease from leaching out was 6 hours.

### Comparative Example 3

Tablets were made as in Example 17, except for using alpha-starch (Matsutani Chemical Industry Co., Ltd.) instead of the polyalkylene polyol derivative. The leach-out test showed that the leaching out of the ingredient ceased in about 15 minutes, failing to provide the desired sustained release preparation.

### Example 19

Sized granules of the release sustaining composition were prepared in the same manner as in Example 5 except that 100 g of a 3.0 wt% aqueous solution of the water-soluble cellulose ether was added to 7 g of the water-soluble polyalkylene polyol derivative to mix them, followed by adding and mixing 50 g of bentonite (Kunibond, Kunimine Industries Co., Ltd.) thereto.

In the same manner as in Examples 9 to 16, the granules thus obtained were dipped in a vitamin C liquid for 1 hour, and dried in a dryer at 40°C, obtaining dry vitamin C-containing granules. The time it takes the yellow color to cease from changing was measured. As the result, the time was 8 hours, and sustained release was confirmed.

## Claims

1. A release sustaining composition comprising a polyalkylene polyol derivative comprising repeating units U-1 having the general formula (1) and repeating units U-2 having the general formula (2) in a molar ratio of U-1/U-2 between 0.5/0.5 and 0.99/0.01, 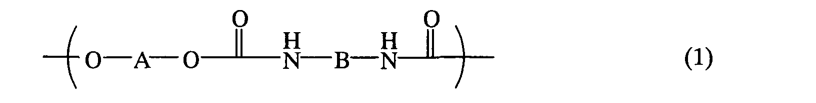 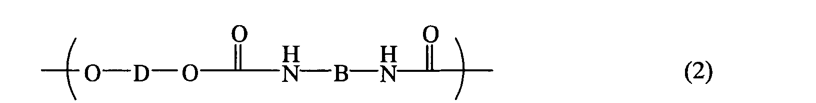 wherein A is a divalent polyoxyalkylene residue derived from a water-soluble polyalkylene polyol, represented by HO-A-OH, having hydroxyl groups at least at both ends and a number average molecular weight of 400 to 100,000; B is a divalent organic residue derived from a polyisocyanate, represented by OCN-B-NCO, having 3 to 18 carbon atoms in total; and D is a divalent organic residue derived from a dihydroxy compound selected from the following general formulae (3) to (5): 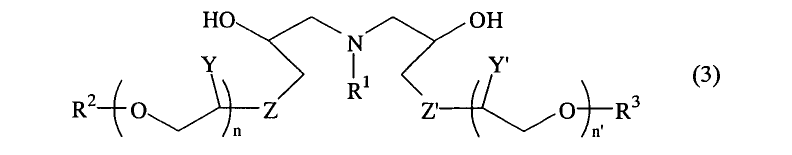 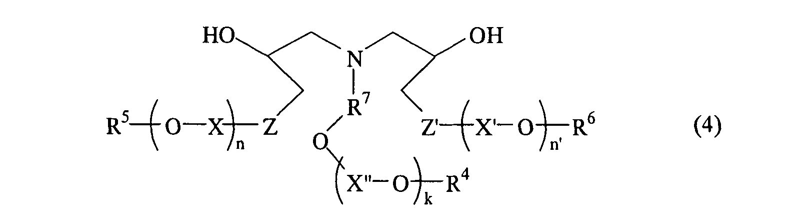 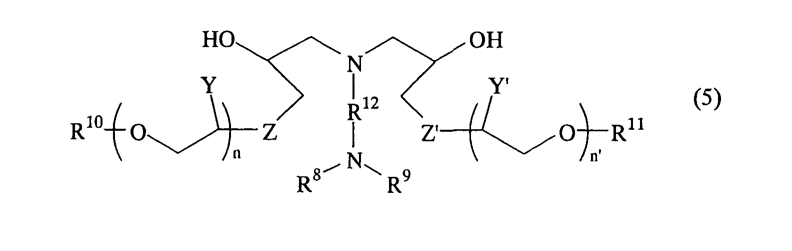 wherein each of R¹ and R⁴ is an unsubstituted or halo-substituted monovalent C₁-C₂₀ hydrocarbon group, each of R², R³, R⁵ and R⁶, which may be the same or different, is an unsubstituted or halo-substituted monovalent C₄-C₂₁ hydrocarbon group, each of Y and Y'
is hydrogen, methyl or CHCl₂, each of Z and Z' is an oxygen atom, sulfur atom or CH₂ group, n is an integer of 0 to 15 when Z is an oxygen atom and equal to 0 when Z is a sulfur atom or CH₂ group, n' is an integer of 0 to 15 when Z' is an oxygen atom and equal to 0 when Z' is a sulfur atom or CH₂ group, n and n' may be the same or different, each of X, X' and X", which may be the same or different, is a C₂-C₁₀ alkylene group, R⁷ is a C₂-C₁₀ alkylene group, k is an integer of 0 to 15, R⁸ and R⁹, which may be the same or different, are monovalent hydrocarbon groups, with a combined number of carbon atoms being in a range of 2 to 20, each of R¹⁰ and R¹¹, which may be the same or different, is a monovalent C₄-C₂₁ hydrocarbon group, and R¹² is a C₂-C₇ alkylene group.

2. The release sustaining composition of claim 1, wherein the dihydroxy compound having the general formula (3) or (4) is a dihydroxy compound having the general formula (6) or (7): 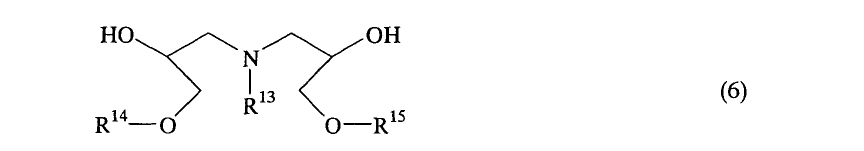 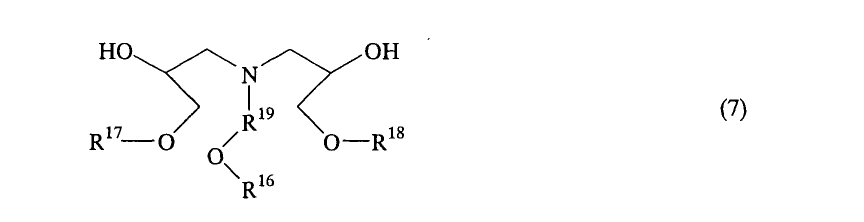 wherein R¹³ is a straight or branched C₄-C₁₈ alkyl group, each of R¹⁴ and R¹⁵, which may be the same or different, is a straight or branched C₄-C₁₈ alkyl group, R¹⁶ is a C₁-C₁₈ alkyl group, each of R¹⁷ and R¹⁸ which are the same is an unsubstituted or halo-substituted monovalent C₄-C₂₁ hydrocarbon group, and R¹⁹ is a 1,2-ethylene, 1,3-propylene or 1,4-butylene group.

3. The release sustaining composition of claim 1 or 2, further comprising a water-soluble cellulose ether.

4. The release sustaining composition of claim 3, wherein said water-soluble cellulose ether is an alkyl cellulose or hydroxyalkyl alkyl cellulose.

5. A sustained release preparation using the release sustaining composition of any one of claims 1 to 4.

6. A preparation according to claim 5 which is a solid particulate incorporating an active ingredient whose release rate is to be controlled.

7. The use, in or as a release-rate controlling composition in relation to an active ingredient whose release rate is to be controlled, of a polyalkylene polyol derivative as defined in claim 1 or 2.
